# EUROPEAN PATENT APPLICATION

(11) **EP 3 132 692 A1**
(43) Date of publication of application: **22.02.2017**
(21) Application number: 15000857.1
(22) Date of filing: 24.03.2015
(51) Int. Cl.: A23L 33/105, A61K 31/352, A61K 31/353, A61P 21/06

(54) **Compositions comprising small molecular inhibitors suitable to inhibit and stimulate signaling pathways in a manner leading to prevention of muscle atrophy**

(71) Applicant: Biosens Croatia, 10020 Zagreb (HR)
(72) Inventor: Lelas, Antonio, 10020 Zagreb (HR)
(74) Representative: Demski, Siegfried

(57) **Abstract**

The present invention relates to the field of prevention and treatment of muscle atrophy. More particular, the present invention relates to a composition comprising hesperetin, (-) epicatechin and myricetin. The present invention further relates to the use of said composition as inhibitor and stimulator for signaling pathways.

## Description

The present invention relates to the field of prevention and treatment of muscle atrophy. The invention provides compositions comprising small molecular inhibitors suitable to inhibit and stimulate signaling pathways in a manner leading to prevention of muscle atrophy.

In accordance with the present invention the term "muscle atrophy" is defined as a decrease in the mass of the muscle. It can be a partial or complete wasting away of muscle. When a muscle atrophies, this leads to muscle weakness, since the ability to exert force is related to mass. The causes of muscle atrophy can be various conditions or disorders: ageing, metabolic syndrome, sarcopenia, AIDS, cancer cahexia, liver failure, kidney failure, congestive heart failure, neurodegenerative muscular diseases (Spinal and bulbar muscle atrophy) etc. Muscle is highly underestimated important organ. It is recently established that promotion of muscle synthesis is negatively correlated to inflammation, a major trigger of majority of metabolic diseases. The molecular mechanisms underpinning the loss of skeletal muscle mass and strength associated with insulin resistance are related and remain to be extensively investigated. It has been repeatedly demonstrated that muscle mass is inversely associated with amount of adipose tissue. Interestingly, adipose tissue is associated with increased risk for cardiovascular diseases and higher incidence of cancer. This means that promotion of muscle synthesis is crucial for maintenance of health, particularly in aged men.

In accordance with the present invention the term "hesperidin" is defined a flavanone glycoside named after the term "Hesperidium", referring to citrus fruits which are the main source of hesperidin. Hesperidin and its aglycone hesperetin are common dietary flavonoids due to being large compounds of citrus fruits (alongside naringenin) and especially the peels and pericarp. Hesperidin has once been noted to comprise up to half of the flavonoid intake of Finland in surveys due to its prominence in the diet (at 28.3mg daily calculated as aglycones) and it could be argued that it is a Traditional Chinese Medicine (perhaps alongside naringenin) since the dried peels of citrus have been used medicinally and referred to as Chimpi. As a glycoside (with sugars), it has an aglycone (without sugars) flavanone known as hesperetin (5,7,3'-trihydroxy-4'-methoxyflavanone) and can also be referred to chemically as hesperetin-7-O-rutinoside (as the sugar it is bound to is rutinose) or hesperetin-7-O-rhamnosyl(1-6)glucoside (since a 'rutinose' sugar is 6-O-α-L-rhamnosyl-D-glucose, or simply a rhamnose sugar.bound to a glucose).

Hesperetin (100µM) has been noted to approximately halve the ability of a ligand to associate with the TGFβ-II receptor and thus to approximately halve the dimerization of these receptors (occurs when a ligand binds and is required for signaling). This reduced receptor and ligand association resultes in partial inhibition of Smad3 phosphorylation and nuclear translocation which is phosphorylated by this receptor. When investigating the inhibitory activities of hesperetin on phosphodiesterase (PDE) enzymes, it has been noted to have inhibitory actions on the PDE4 isomer (IC50 of 28.2+/-1.1µM). After absorption, hesperidin can be metabolized by UGT enzymes to form hesperetin glucuronides and it is near completely metabolized with up to 87% being found as hesperetin monoglucuronide and 13% as sulphoglucuronides. The glucuronides formed include 3'-O-glucuronide, 7-O-glucuronide, 5,7-O-diglucuronide, and 3',7-O-diglucuronide while the sulphogluduronide is thought to refer to Hesperitin-3'-O-sulphate. Consumption of hesperidin (via orange juice) alongside 150 mL yogurt has been noted to reduce the Cmax of the main metabolite (hesperitin-7-O-glucuronide) by 29% and overall exposure (AUC) by 27%, but overall hesperetin absorption was not changed; this was thought to be due to relative increases in other glucuronidation metabolites. Hesperetin glucuronides are still metabolically active, particularly hesperetin-7-O-glucuronide which seems to be the primary metabolite in humans as the other known one (hesperetin-3-O-glucuronide) has only been detected in rats so far. The potency of hesperetin-7-O-glucuronide relative to hesperetin is similar or mildly less potent.

Hesperetin has been noted to stimulate PPARγ and PPARα activity in a concentration dependent manner between 20-160 µM; secondary to this, hesperetin could induce secretion of adiponectin from 3T3-L1 adipocytes with 80µM hesperidin having similar potency to 10µM rosiglitazone

In isolated myoblast C2C12 cells, hesperidin (100µM) is able to promote myocyte differentiation in low mitogen medium associated with more activity of myogenin, a muscle cell growth factor downstream of MyoD, and MCK without any influence on either MyoD nor MEF2C transcription. It appeared that hesperidin enhanced MyoD actions on the genome (not influencing the Interaction of MyoD and MEF2C), resulting in enhanced transcription of myogenin and MCK. In mice subjected to freeze injury (tibialis) followed by hesperidin injections at 50mg/kg for six days, muscle regenerative capabilities appeared to be significantly enhanced relative to control.

In accordance with the present invention the term "myostatin" is defined as a negative regulator of muscle mass, also known as growth and differentiation factor 8 (GDF8), which is a member of transforming growth factor (TGF)-ß superfamily, which has been found to be capable of negatively affect (inhibit) muscle synthesis (catabolic), thus modulating the body weight and muscle composition in laboratory and farm animals.

Its inhibition became an interesting approach towards achieving a more constant anabolic state. After release from cell and its pro-peptide, myostatin commences catabolic signal by docking to activin type II receptor B (ACTRIIB). This induces downstream cascade events which consequently activate atrophy related genes and proteins.

In accordance with the present invention, the term "myricetin" is defined as a member of the flavonoid class of polyphenolic compounds with antioxidant properties. It is commonly derived from fruits, vegetable, nuts and herbs such as Blueberry leaves, Oranges, Sea Buckthorn, Chia seeds (Salvia hispanica), Japanese raisin tree (Hovenia dulcis), Carob extract (Ceratonia siliqua), Pistachio, Grape Seed Extract, Cabbage, Peppers Garlic and others. Myricetin is structurally similar to fisetin, luteolin, and quercetin and is reported to have many of the same functions as these other members of the flavonol class of flavonoids.

Myricetin has been noted to prolong average (18%) and maximal (21.7%) lifespan in C. Elegans, which was a potency greater than other tested flavanols (quercetin, kaempferol, and naringenin) associated with reducing oxidative damage to the mitochondria and proteins. When tested in mev-1(kn1) mutants, (reduced lifespan associated with higher mitochondrial oxidative stress) all flavonoids reduced mitochondrial oxidative stress, yet only myricetin increased average lifespan (16%) in these mutants. Myricetin showed the scavenging effect of 1,1-diphenyl-2-picrylhydrazyl (DPPH) radicals on intracellular ROS. In addition, myricetin restored the activity and protein expression of cellular antioxidant defense enzymes such as superoxide dismutase (SOD), catalase (CAT), and glutathione peroxidase (GPx) reduced by hydrogen peroxide (H₂O₂) treatment. H₂O₂-induced cellular DNA and lipid damages, and myricetin was found to prevent the DNA damage shown by inhibition of DNA tail and it decreased nuclear phospho-histone H2A.X expression, which are both markers for DNA strand breakage. Myricetin also mediates survival of H₂O₂ induced cell death via interfering MAPK signalling pathway. Myricetin has been reported to have anti-inflammatory activity in vitro. Myricetin reduces secretion of TNF-alpha and IL-1ß in lipopolysaccharide (LPS)-stimulated RAW264.7 cells. Myricetin inhibits LPS-induced production of IL-12 by downregulating transcription factor nuclear factor kappa-B (NF-kB) binding activity in RAW264.7 cells. In addition, myricetin decreases IL-1ß-induced production of IL-6 in synovial cells. Having such anti-inflammatory activity, more implications for beneficial activity of myricetin were examined.

Findings suggested that myricetin may have a protective effect against diet-induced obesity and insulin resistance in mice fed HFHS diet, and that alleviation of insulin resistance could partly occur by improving obesity and reducing serum proinflammatory cytokine levels. Results show that myricetin increased the antioxidant levels in plasma, erythrocyte lysate, and breast tissue and was effective in preventing the oxidative damage induced by the carcinogen DMBA (7,12-dimethylbenzanthracene), Myricetin 50, 100, and 200 mg/kg/oral for 120 days treated animal resulted comparable results to that of standard vincristine and control groups, which gives indication of chemopreventive effect. Moreover, myricetin induces cytotoxicity and DNA condensation in human colon cancer cells via promoting the release of apoptosis-inducing factor from mitochondria (caspase-3 and -9). Finally myricetin was able to inhibit human DNA topoisomerase II (topo II) activity with an IC50 value of 27.5 µM of 16 polyphenols tested and UVB-induced angiogenesis by targeting Raf kinase/PI3K.

In accordance with the present invention the term "(-) epicatechin" (EPI) is defined as a flavan-3-ol, a type of natural phenol and antioxidant. It is a plant secondary metabolite. It belongs to the group of flavan-3-ols (or simply flavanols), part of the chemical family of flavonoids. Beverages, such as, green tea and cocoa - derived products are a source of EPI. Research show that mice fed for 15 days with EPI had improved exercise performance accompanied by: (1) an increased number of capillaries in the hindlimb muscle; and (2) an increased amount of muscle mitochondria as well as signaling for mitochondrial biogenesis. These results suggest that EPI increases the capacity for muscle aerobic metabolism, thereby delaying the onset of fatigue. Human ingestion of flavanol-rich cocoa was associated with acute elevations in levels of circulating NO species, an enhanced FMD response of conduit arteries, and an augmented microcirculation. In addition, the concentrations and the chemical profiles of circulating flavanol metabolites were determined, and multivariate regression analyses identified (-)-epicatechin and its metabolite, epicatechin-7-O-glucuronide, as independent predictors of the effects after flavanol-rich cocoa or tea ingestion.

EPI also improves mitochondrial-related protein levels and ameliorates oxidative stress in dystrophic δ-sarcoglycan null mouse striated muscle. This was demonstrated by significant normalization of total protein carbonylation, recovery of the glutathione/oxidized glutathione ratio and enhanced superoxide dismutase 2, catalase and citrate synthase activities as well as decreases in heart and skeletal muscle fibrosis. In mice, myostatin and senescence-associated β-galactosidase levels increase with aging, while those of follistatin and Myf5 decrease. EPI decreases myostatin and β-galactosidase and increases levels of markers of muscle growth. In humans, myostatin and β-galactosidase increase with aging while follistatin, MyoD and myogenin decrease. Treatment for 7 days in aged men with EPI increases hand grip strength and the ratio of plasma follistatin/myostatin.

In accordance with the present invention the term "fenugreek" is defined as the plant Trigonella foenum-graecum L. Fenugreek seeds and extract has been used historically for various uses such as antispasmodic, appetite stimulant, high cholesterol, wounds, blood cleanser, and expectorant effects. It is derived from both Ayurveda (mostly) and Traditional Chinese Medicine (to a lesser degree) and unlike most traditional medicines. It also appears to be a condiment. There are some other traditional usages of fenugreek seeds including using them in beer as an at-home remedy for anxiety and hopelessness (Danish). Fenugreek seeds; the main active component of this plant primarily contains;
1. Trigonelline
2. 4-hydroxyisoleucine
3. Protodioscin similar to Tribulus terrestris
4. The two Trigoneosides, Trigofaenoside A and Glycoside D
5. Diosgeninand Yamogenin

In streptozotocin-induced diabetic rats, 1,500mg/kg of fenugreek seeds daily for 21 days was associated with a 58% reduction in blood glucose levels (still 116% higher than true control) with no significant influence on insulin. Reduction in blood glucose have also been noted in Alloxan-induced diabetic rats (2-8g/kg of the seeds) 21 days of 1,500mg/kg Fenugreek is associated with improvements in the hepatic enzymes G6P (59% of the change seen via diabetes was reversed) and G6P dehydrogenase (30%) as well as improving hepatic glycogen storage; phosphofructokinase, reduced during diabetes, was unaffected. A beneficial alteration of hepatic enzymes has also been noted in Alloxan-induced diabetic rats in both the kidneys and liver (5% of the rat diet). Fenugreek appears to hold some promise in reducing biochemical markers of diabetes in diabetic rats. One study using high doses of Fenugreek (2-8g daily) in alloxan induced diabetic rats noted that the healthy control rats also experienced a reduction in blood glucose. This appears to be relevant in humans with Type I diabetes (100 g defatted Fenugreek) as well as Type II diabetics, where a 53 % increase in insulin sensitivity was noted via HOMA relative to the control group using 1 g daily of a hydroalcoholic extract. Benefits for glycemic control have been seen with the seeds themselves, de-fatted seed extract and whole seed powder. Fenugreek has been successfully incorporated into a bread product at 5% fenugreek flour by weight without exerting adverse taste effects onto the bread, this has been used in vivo to lower the glucose spikes after eating the bread in diabetics. When administered at 900 mg and in conjunction with 3.5 g Creatine monohydrate, fenugreek+creatine appears to be as effective at increasing lean body mass and strength over 8 weeks as creatine alongside 70 g dextrose. It was noticed that compound 4-Hydroxyisoleucine from Fenugreek seeds (seems like one of most important active principles of Fenugreek) stimulates glucose uptake by increasing surface GLUT4 level in skeletal muscle cells via phosphatidylinositol - 3 - kinase - dependent pathway and also inhibits palmitate-induced, ROS-associated inflammation and restores insulin sensitivity through regulating IRS-1 function in skeletal muscle cells.

In accordance with the present invention the term "excipient" is defined as a substance that is present alongside the active ingredients and added to the active ingredients to a give suitable consistency or form. Thus, excipients are inactive ingredients that do not increase or affect the therapeutic action of the active ingredients.

In accordance with the present invention the term "hypromellose" is defined as hydroxypropyl methylcellulose (HPMC), which is a semisynthetic, inert and viscoelastic polymer that is often used as excipient.

In accordance with the present invention the term "active ingredient" is defined as the chemically active substance, which is meant to produce the desired effect.

In accordance with the present invention the term "per weight" refers to weight of the dried component the term "per weight" refers to.

In accordance with the present invention the term "dried" is defined as having a water content of less than 5 %. Drying can be achieved by exposure of the components, e.g. the plant derived active ingredients, to a temperature above 50 °C, preferably between 50 and 80 °C. for at least 3 h or by extraction process including a drying step as described above or including a step of solvent evaporation.

In accordance with the present invention the term "extract" is defined as one or more substances being separated from raw material by extraction process. The extraction process can be carried out by using extraction methods known from prior art, preferably solvent extraction methods, e.g. by using polar protic and polar aprotic solvents, non-polar solvents as well as CO₂ extraction, depending on the active ingredient. Furthermore, it is possible to increase the amount of an active ingredient in the extract by drying or extraction. The amount of a component, e.g. an active ingredient, can be determined primarily via HPLC.

Skeletal muscle is the most abundant tissue in the body of vertebrates and is involved in many different important functions. For example, skeletal muscle is the primary target of glucose uptake, can regenerate after injury, and is subjected to size remodeling. Overall, skeletal muscle mass embodies strength, endurance, and physical performance. Decreased muscle mass or muscle atrophy appears when protein degradation exceeds protein synthesis, leading to shrinkage of the cross-sectional area of myofibers and decreased muscle strength. Muscle atrophy is a physiological consequence of aging (i.e., age-related sarcopenia), defined as the presence of both low muscle mass and low muscle function (strength or performance), but it may also result from prolonged periods of rest or a sedentary lifestyle. Moreover, muscle atrophy represents a clinical feature of cachexia, a syndrome designating reduced life expectancy and accompanying many illnesses like chronic heart failure (CHF), chronic obstructive pulmonary disease (COPD), chronic kidney disease (CKD), cancer, HIV, sepsis, immune disorders, and dystrophies.

Among the different pathways controlling muscle size, the PI3K/Akt pathway is central and is mainly initiated upon IGF-1/insulin stimuli to promote protein synthesis and to block degradation. In the cytoplasm, Akt1 activates S6 kinase 1 (S6K1) via mammalian target of rapamycin (mTOR), which leads to increased protein synthesis. Akt1 phosphorylates also FoxO transcription factors, specifically making FoxO3 unable to enter the nucleus and to drive transcription of certain ubiquitin ligases (e.g., muscle atrophy F-Box (MAFbx/Atrogin-1) and muscle RING finger-1 (MuRF1)) or autophagy-related genes (e.g., LC3 and Bnip3), which account for the protein breakdown settled by the ubiquitin-proteasomal and autophagic/lysosomal pathways.

Activin type II receptors (ActRIIA/ActRIIB) mediate the signaling of a subset of transforming growth factor-β (TGF-β) ligands, including activin A, activin B, myostatin, and GDF-11. Protein ligand binding to ActRIIA/IIB leads to the activation of type I receptors (ALK4, 5, or 7), which initiate an intracellular signaling cascade centered on Smad2/3 transcription factors. Smad2/3 activation drives the expression of genes involved in cellular proliferation, differentiation, apoptosis and extracellular matrix deposition, and is critical for the maintenance of adult tissue homeostasis. Most interesting pathways represent myostatin/ActRIIB/SMAD and activin/ActRIIA/SMAD which are responsible for major catabolic signaling cascade.

Notably, disruption of Akt1 via major catabolic pathway; myostatin/ActRIIB/Smad results in muscle wasting as a consequence of activation of previously mentioned autophagy related genes. myostatin (produced primarily in skeletal muscle) binds to its receptor complex ActRIIB/Alk 4 or 5 on skeletal muscle, resulting in activation of the Smad 2/3, mitogen-activated protein kinase and inhibition of the PI3K intracellular signaling pathways that together result in gene transcriptional changes (MuRF1, Atrogin-1) and effects on protein synthesis that ultimately give rise to muscle atrophy. Additionally, SMAD 3 directly amplifies FoxO3-induced MuRF-1 promoter activity. Consequently, myostatin/ActRIIb and PI3K/Akt interaction and activation emerged as therapeutic targets yielding various intervention approaches.

The majority of these approaches act extracellularly to block myostatin engaging with the ActRIIB/Alk4/5 receptor complex, either by binding directly to myostatin (Fstl3, follistatin, myostatin antibody, GASP1, myostatin propeptide, decorin peptides, ActRIIB-Fc) itself or by binding its receptor complex (ActRIIB antibody) in order to block myostatin engaging its receptor complex and activating downstream signaling. Some of the inhibitors are naturally occurring (myostatin propeptide, Gasp1, follistatin, Fstl3) whereas others are engineered (myostatin antibody, ActRIIB antibody, ActRIIB-Fc).

There is limited amount of data for small molecule inhibitors of myostatin/ActRIIB pathway. Although development of antibody production and gene delivery methods offer high selectivity and good binding affinity towards desired target, industrial production of antibodies and recombinant proteins is expensive. Peptides, antibodies and proteins also suffer from adequate delivery method (injections). On the other side, small molecule inhibitors offer ease of production and application. It will even be more advantageous, if the small molecule inhibitors are derived from plants or plant fractions. This is due to the fact that natural products are often easy to obtain and are already used in diverse applications for many years, e.g. in traditional Chinese medicine so that possible side effects are usually known.

Literature argues several strategies representing viable approaches to disrupt/ameliorate myostatin signaling. Application of histone deacetylase (HDAC) inhibitor trichostatin A, for example increased production of follistatin and enhanced expression of markers of regeneration following muscle injury. In similar fashion, inhibition of HDAC and DNA methyltransferase by sulforaphane represses myostatin gene (MSTN) expression, accompanied by strongly attenuated expression of negative feedback inhibitors of the MSTN signaling pathway, which was confirmed in porcine satellite muscle cells. This inevitable argues that small molecule epigenetic targeting approach is attractive method for myostatin signaling interruption.

Furthermore, it is known that beta-agonist formoterol significantly increase levels of various follistatin isoforms and significantly decrease the expression levels of the myostatin receptor. However, human effective doses may cause serious adverse effects characteristic for formoterol. Nonetheless, it seems that beta adrenergic agonists have a perspective as far as muscle preservation or muscle growth is concerned. It is further known that S-pindolol and derivatives can significantly enhance muscle mass.

Furthermore, it is possible to interact with the myostatin/ActRIIB pathway by using a small molecule inhibitor of myostatin protein itself. By means of structure based approaches, the myostatin binding region can be proposed (most likely interfering myostatin/ActRIIB binding) and confirmed in silico and in vitro. It was confirmed that suspected kinase region of Activin receptor A (ActRIIA) inhibition (intracellular portion of receptor) suspends myostatin signalling. This was achieved with Dorsomorphine and LDN-193189 molecule followed by pronounced myoblast differentiation.

It is further known that natural flavanol (-)-epicatechin treatment for 7 days increases hand grip strength and the ratio of plasma follistatin/myostatin in mice, thus being an interesting strategy towards treatment of sarcopenia.

A few small molecule inhibitors of myostatin signaling or decreasing myostatin levels have been found so far:
1. (S)-Pindolol, (S)-propanol, tertalol, or bopindolol
2. Sulforaphane
3. Dorsomorpine and LDN-193189
4. EGCG (epigallocatechin-3-gallate)
5. (-)-Epicatechin

There is a considerable amount of data for PI3K/Akt pathway stimulators for muscle growth. Accordingly, leucine and its derivative HMB are known to stimulate PI3KIAkt/mTOR pathway resulting in increased muscular mass provided that sufficient protein intake is enabled. Phosphatidic acid (PA) is implied in direct mTOR stimulation, a part of Akt related pathway, which results in muscle growth. Another interesting approach comes from PI3K/Akt stimulation via IGF1 and insulin receptor sensitization. Ursolic Acid is known to stimulate muscle growth via sensitization of insulin and IGF1 receptor through PTP1 B inhibition and Akt signaling resulting in increased glucose uptake by muscle. Additionally, an increasing uptake of glucose to muscle cells due to increasing glucose transporter 4 (GLUT4) surface and PI3K/Akt signaling was reported for 4-hydroxyisoleucine (4-HIL).

In order to overcome the above mentioned drawbacks in the art, ActRIIA and ActRIIB were computationally targeted with natural small molecules belonging to flavanone and flavonol groups to inhibit intracellular kinase domain signaling. Natural products and their corresponding active molecules have historically been a rich source of lead molecules in active ingredients discovery. However, most of natural origin candidates or "lead" molecules have unspecific binding properties and require higher concentration for activity. Therefore lead molecule optimization is often employed as a tool to synthesize-produce more potent compounds. Instead, small natural molecule sets (flavanones and flavonols) with known bioavailability properties were chosen and those properties were combined in order to generate a potent formulation. The hypotheses were checked via *in silico* screenings. Docking of these molecules was performed to ActRIIA and ActRIIB regions that are believed to be crucial for SMAD signaling interruption.

Briefly, several conformations of intracellular kinase region for both protein ActRIIA and ActRIIB were used. For ActRIIA x-ray structure pdb files PDBID 4ASX, 3SOC and 3Q4T with known potent cocrystallized inhibitors beta-carboline, quinazolin and dorsomorphin were chosen. All 3 were prepared with protein preparation wizard (Schrodinger Suite), assigned with missing side chains, hydrogens and ionization states, followed by pKa values and 100 step minimization via OPLS 2005 force field. Cocrystalized ligands were assigned with binding states with lowest energy penalty. Same ligands were used as centroids for Glide grid generation with appropriate hydrogen bonds constraints important for ligand binding. Additionally 3SOC structure was used to create apo structure (ligand free) and to run molecular dynamics simulation (Desmond) to create more relevant protein conformations. Simulation was run over period of 50 ns. Trajectory cluster script (Schrodinger Suite) was used to Yield 8 different protein conformations. Two of the most best representatives were selected to join previously prepared three structures in cross docking against "in house" library of 35 selected flavonols. Ligands were prepared (minimized, assigned with force field, tautomeric and ionization states) via LigPrep (Schrodinger Suite) in pH 6.9-7.9. Docking against five representative ACTRIIA conformation with respective hydrogen bonds constrains was established with Virtual screening workflow (Schrodinger Suite) which included Glide XP followed by Prime MMGBSA (Molecular Mechanics/Generalized Born Surface Area calculation method by Schrodinger Suite) with VSGB solvation model and residue flexibility of 4 angstroms around ligand; in order to approximate relative binding affinities

ActRIIB kinase domain PDBID 2QLU was prepared in same fashion described above by Protein preparation wizard with default values. For testing the binding pocket hypothesis, molecular structures of above mentioned three ActRIIA kinase domains co-crystalllzed were superimposed with ligands to match the binding pocket, SiteMap (binding pocket determination) software was used to verify whether selected pocket is plausible. More specifically, "hinge" region of protein pocket was targeted, characteristic for kinase specific domain. In order to get more conformations of the protein molecular dynamic simulation was ran as described above. Five representative conformations were selected for cross docking via Virtual screening workflow. The binding sites were generated by sitemap and docked against 27 selected natural flavanones. The docking processes, assumed also GlideXP Prime MMGBSA (Schrodinger Suite) with VSGB solvation model and residue flexibility of 4 angstroms around ligand; in order to approximate relative binding affinities of congeneric series of ligands (sharing a common core).

In both cases, Molecular dynamics software Desmond (Schrodinger Suite) was used to verify and visually observe ligand stability within the binding pocket. Simulations were set to 30 nanoseconds. Ligand movement, protein residue side-chain movements, hydrogen bonds and Pi-Pi interactions were accounted during stability observation.

Myricetin showed best binding intracellular kinase domain of ActRIIA of -12.8 kcal/mol, while hesperetin shows high score binding to intracellular kinase domain of ActRIIB of -11.6 kcal/mol. Calculated relative binding energy (MMGBSA ranking protocol) for myricetin and hesperetin were -83.037 kcal/mol and -72.108 kcal/mol, respectively. These values were in agreement with docking results ranking. In order to further test binding, we ran molecular dynamics simulation of 30 ns for each bound system to check whether such docked molecule conserve initial binding position in the protein pocket region. Myricetin was stable with minimal position change over simulation (small enthalpic and bigger entropic contribution) and initial 5 hydrogen bonds tuned into 6 from 15^{th} ns (see figures 1 and 2). Hesperetin also stays firmly buried in the binding site, establishing additional hydrogen bond interaction (from 4 to 5), and achieving even stronger, more fixed position over simulation course (see figures 3 and 4).

It was surprisingly found that the active ingredients hesperetin, (-) epicatechin and myricetin act synergistically. Thus, a combination of these components leads to a composition showing an increased effect on prevention of muscle atrophy compared to one of the components alone in an amount equal to the sum of the components of the composition. Thus, the present invention relates to a composition comprising hesperetin, (-) epicatechin and myricetin. It was found that a composition comprising hesperetin, (-) epicatechin and myricetin is able to overcome the above mentioned drawbacks in the art and prevents muscle atrophy, improves muscle growth, in particular the lean muscle mass and improves the utilization of muscle glucose and fat. Said composition further supports adipose tissue reduction, supports healthy sugar levels in blood, supports healthy muscle function, prevents muscle catabolic processes, reduces fatigue and promotes vigilance.

In a preferred embodiment, the present invention relates to a composition comprising 15-66%, preferably 20-50 %, more preferably 25-40 % per weight hesperetin, 15-66%, preferably 20-50 %, more preferably 25-40 % per weight (-) epicatechin and 5-66%, preferably 7-25 %, more preferably 10-20 % per weight myricetin.

Preferably, the composition comprises 15-66%, preferably 20-50 %, more preferably 25-40 % per weight hesperetin, 15-66% %, preferably 20-50 %, more preferably 25-40 % per weight (-)-epicatechin, 5-66%, preferably 7-25 %, more preferably 10-20 % per weight myricetin and 0,5-20%, preferably 1-10 %, more preferably 1,5-5 % per weight 4-hydroxyisoleucine. Said composition further improves PI3K/Akt stimulation leading to further prevention of muscle atrophy and improvement of muscle growth.

The composition according to the present invention may comprise the active ingredients in form of precursors that can be metabolized into the active ingredients by human metabolism. The precursors have to be present in an amount that leads to the metabolized product in form of hesperetin, (-) epicatechin or myricetin in the amount as mentioned above.

In a preferred embodiment, the active ingredients of the compositions according to the invention are homogenized and mixed with one or more excipients. The mixed composition may be formed as a capsule or pressed in tablet form.

As excipients preferably binders and/or coatings are used. Saccharides and their derivatives, proteins, synthetic polymers and further binders known from the prior art, which hold the ingredients in a tablet together can be used as binders. Hypromellose, synthetic polymers, shellac, corn protein zein or other polysaccharides and further coatings known from the prior art, which protect the ingredients can be used as tablet coatings. Preferred excipients are hypromellose, methylcellulose, phosphatidylserine or phosphatidylcholine.

The present invention also relates to a composition consisting of hesperetin, (-) epicatechin, myricetin and possibly 4-hydroxyisoleucin within the concentration ranges mentioned before and one or more excipients.

In form of capsules or tablets, the compositions according to the present invention can be easily taken orally, e.g. as a food supplement. However, the compositions may also be added to diets (drink or food). Forms and properties of the food and drink are not particularly limited so long as the active ingredients are not degraded. Thus, the compositions may be administered as a part of dietary supplement or nutritional product.

Even more preferably, compositions according to the present invention comprise ingredients, which are derived at least in part from plants or plant fractions, preferably, which are completely derived from plants or plant fractions. In particular, the composition consists of ingredients derived from plants or plant fractions.

It is advantageous when the plant or plant factions contain 10-100 % per weight of the desired ingredient.

Preferably, the compositions according to the present invention comprise hesperetin at least in part, preferably completely, in form of orange extract (Citrus aurantium) and/or (-) epicatechin at least in part, preferably completely, in form of green tea extract (Camellia sinesis) and/or (-) epicatechin at least in part, preferably completely, in form of cocoa extract and/or myricetin at least in part, preferably completely, in form of wild bayberry extract and/or the optional 4-hydroxyisoleucine at least in part, preferably completely, in form of fenugreek extract.

The compositions according to the present invention may comprise the precursor that can be metabolized into hesperetin by human metabolism, e.g. hesperidin, at least in part, preferably completely, in form of orange extract (Citrus aurantium) and/or the precursor that can be metabolized into (-) epicatechin by human metabolism at least in part, preferably completely, in form of green tea extract (Camellia sinesis) and/or cocoa extract, and/or the precursor that can be metabolized into myricetin by human metabolism at least in part, preferably completely, in form of wild bayberry extract.

The orange extract (Citrus aurantium) being used in a composition according to the present invention preferably contains 30-100%, more preferably 50-95 %, even more preferably 75-90 % per weight hesperidin.

The green tea extract (Camellia sinesis) and the cocoa extract being used in a composition according to the present invention preferably contain 30-100%, more preferably 50-95 %, even more preferably 75-90 % per weight (-) epicatechin.

The wild bayberry extract being used in a composition according to the present invention preferably contain 25 - 100 %, more preferably to 40 - 60 %, even more preferably to 45-55 % per weight myricetin.

The fenugreek extract being used in a composition according to the present invention preferably contains 1 - 100 %, more preferably 10 - 60 %, even more preferably 15 - 25 % per weight 4-hydroxyisoleucine.

It is advantageous when the composition additionally comprises nutrients, in particular magnesium chloride and/or magnesium citrate and/or magnesium lactate and/or vitamin D3.

It is advantageous when the composition additionally comprises one or more flavonoids or its derivatives, preferably selected from the group consisting of kaempferol, leucodelphinidin, poncirin, diosmetin, taxifolin, dihydromyricetin, epigallocatechin-3-o-gallate, and/or genistein.

Preferably, the compositions according to present invention may additionally contain further components, in particular silk peptide and/or leucine and/or its metabolite β-hydroxy-β-methylbutyric acid and/or piperine.

The present invention further relates a method for preventing muscle atrophy along with improving muscle growth, in particular the lean muscle mass, improving the utilization of muscle glucose and fat. Said method further supports adipose tissue reduction, supports healthy sugar levels in blood, supports healthy muscle function, prevents muscle catabolic processes, reduces fatigue and promotes vigilance. Said method for preventing muscle atrophy in a subject comprises the step of administering to said subject a composition according to the present invention as described above.

Preferably, the composition is administered orally to the subject. Preferably, the dosage of the composition per administration is 0,3 - 2 g. The dosage of each of the active ingredients per administration is preferably between 50-300 mg. Preferably, the composition is administered twice a day. It is advantageous when the composition is administered within 2 hours before sporting activity.

The present invention also relates to use of the composition according to the present invention as described above as inhibitor for myostatin/ActRIIB signaling pathway as well as stimulator for PI3K/Akt signaling pathway.

Further advantagous embodiments of the invention result from the sub-claims.

The invention is further explained with the aid of the figures.

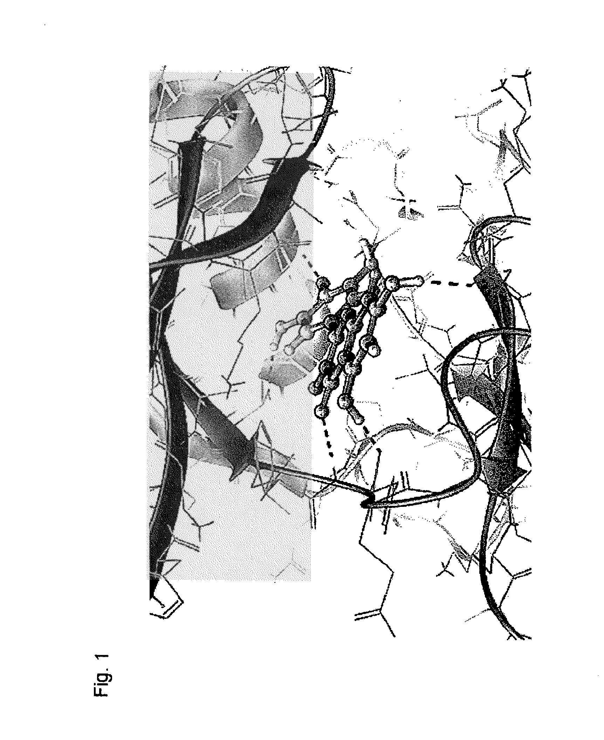

It shows
- Fig. 1: Myricetin in initial bound state with ActRIIA docked via Glide XP and refined by MMGBSA,

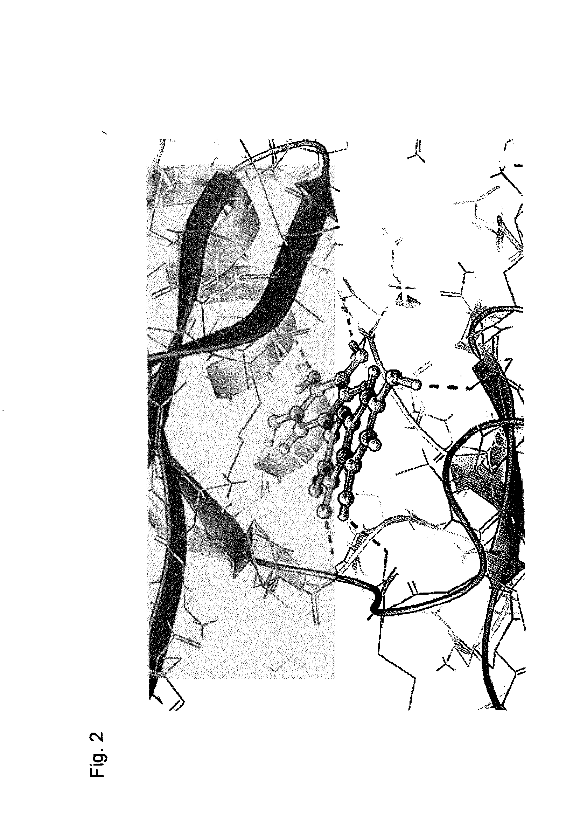

- Fig. 2: Myricetin in bound state with ActRIIA after 20 nanoseconds of MD simulation,

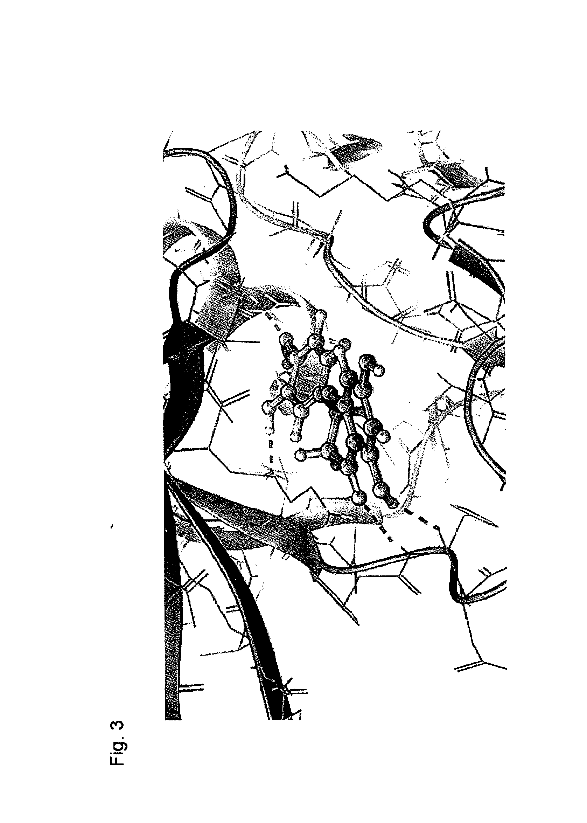

- Fig. 3: Hesperetin in initial bound state with ActRIIB docked via Glide XP and refined by MMGBSA, and

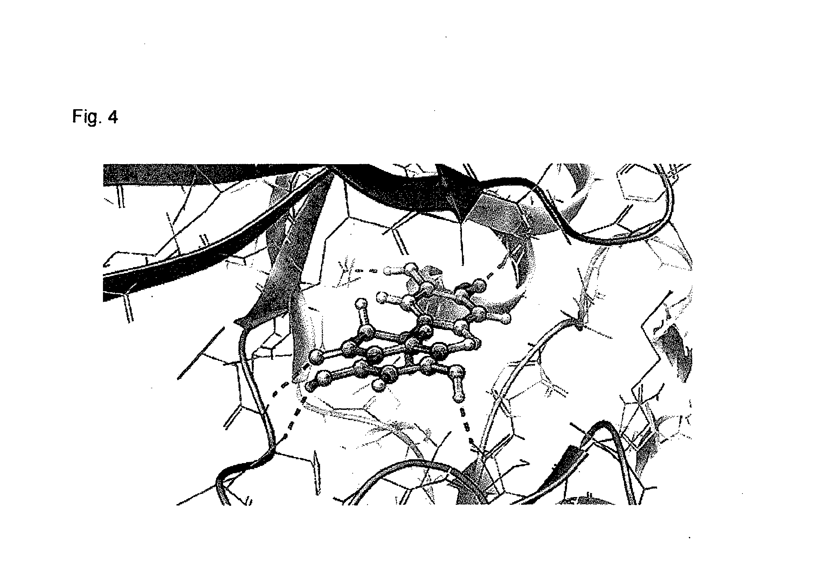

- Fig. 4: Hesperetin in bound state with ActRIIB after 20 nanoseconds of MD simulation.

### Example

In the efficacy study, 18 resistance trained men with at least 5 years of experience in weight lifting voluntarily participated. Participants were divided in 3 equal groups; receiving placebo (hypromellose, 2 times a day), (-)-epicatechin ((EPI), 2x 150 mg a day) and (-)-epicatechin combined with hesperetin and myricetin ((E+H+M), 2x 150+100+50 mg a day). Maximally trained individuals were chosen as further muscle gains in such groups are usually minimal, even with dietary interventions. This is primarily explained due to a fact that such trained individuals reached so called "natural genetic plateau" where muscle tissue works within physiological optimum defined by the genetic blueprints, so even radical interventions (except anabolic steroids) and exercise regime cannot bring significant results. This was confirmed by participants as they had no means to achieve particularly better results over last few years of exercise. All subjects were asked to maintain the same caloric intake during 12 week study 5 days a week exercise. During 2 months study participants were taking 30 g of post exercise amino acid blend (whey, soy and casein protein) with following profile per 100 g:
Alanine 7.1g, arginine 9.2 g, aspartic acid 6.2 g, glutamic acid 21.5 g, glycine 12.2 g, histidine 1.1 g, isoleucine 6.9 g, leucine 11.1 g, methionine 0.3 g, phenylalanine 2.7 g, proline 11.2 g, serine 2.7 g, threonine 1.5 g, tyrosine 0.5 g and valine 5.8 g.

For strength measures at the beginning and the end of study, the subjects were required to perform bench press and squats exercise with their 1-repetition maximum (1-RM). During intermediate period of study, standard routine exercise for all body parts were performed. A 90-s rest period was required between each set, for all exercises that are usually performed. The load for the exercises was self-determined by the subject, but they were required to use a load that allowed them to perform a 10-12 RM. Participants were not allowed to practice 1-repetition maximum (1-RM) bench and squat exercise during study course to avoid biased fast twitch fiber muscle development that yields better explosive strength results. Logs were controlled and provided by gym supervisor personnel.

Body composition was examined at the beginning and the end of study using dual energy x-ray absorptiometry - DEXA scans (DiscoveryTM by Hologic). Total body estimates of fat and non-bone lean tissue were determined according to company supplied algorithms. Muscle thickness for Vastus lateralis and Triceps brachii lateralis was examined at the beginning and the end of study by B-mode ultrasound imaging (Toshiba PowerVision 8000) as the distance between the subcutaneous adipose tissue and inter-muscular belly interface. Total Cretine Kinase as a marker of muscle damage was performed by VITROS® 4600 (Ortho Clinical Diagnostics) at the beginning and the end of study.

**TABLE1:**

| **VARIABLE** | **GROUP** | **BEGGINING** | **END** |
|---|---|---|---|
| 1-RM SQUAT (kg) | PLACEBO | 158±32 | 163±36 |
| | EPI | 153±24 | 170±22 |
| | EPI+H+M | 156±28 | 178±23 |
| 1- RM BENCH PRESS | PLACEBO | 146±19 | 150±21 |
| | EPI | 148±21 | 154t18 |
| | EPI+H+M | 146±19 | 163±16 |
| Vastus Lateralis thickness (mm) | PLACEBO | 24.2±3.7 | 24.3±3.3 |
| | EPI | 23.8±3.9 | 24.6±2.9 |
| | EPI+H+M | 24.3±4.1 | 27.8±3.3 |
| Triceps Brachii Lateralis thickness (mm) | PLACEBO | 30.6±4.3 | 32.1±3.8 |
| | EPI | 30.8±4.4 | 33.8±2.2 |
| | EPI+H+M | 30.8±4.2 | 36.5±2.7 |
| Body mass (kg) | PLACEBO | 94.5±12.2 | 94.1±11.8 |
| | EPI | 93.2±14.6 | 93.8±10.5 |
| | EPI+H+M | 93.8±17.7 | 95.6±12.7 |
| Body fat (kg) | PLACEBO | 10.3±4.7 | 10.2±4.9 |
| | EPI | 11.2±5.3 | 9.1±4.2 |
| | EPI+H+M | 10.8±5.1 | 8.8±2.6 |
| Lean body mass (kg) | PLACEBO | 76.3±4.5 | 77.1±5.6 |
| | EPI | 76.4±5.8 | 77.2±5.8 |
| | EPI+H+M | 75.8±5.3 | 79.7±3.9 |
| Total Creatine Kinase (CK reff range: 38-120 ng/ml) | PLACEBO | 88±21 | 91±19 |
| | EPI | 86±24 | 73±16 |
| | EPI+H+M | 89±22 | 56±19 |

The results clearly demonstrate that a composition comprising hesperetin, myricetin and epicatechin exhibit yet unexpected synergistic effects that could be explained by results received *in silico* showing auxiliary binding with ActRIIA/ActRIIB, which furthermore obstructs myostatin/activin signaling pathway in addition to follistatin enhancement. The unexpected synergy could also be explained by enhanced CaCO₃ transport (intestinal transport) through which hesperetin and myricetin might act as enhancers of (-)-epicatechin bioavailability.

Thus the present invention relates to, but is not limited to:
- A composition comprising hesperetin, (-) epicatechin and myricetin
- Said composition comprising 15-66%, preferably 20-50 %, more preferably 25-40 % per weight hesperetin, 15-66%, preferably 20-50 %, more preferably 25-40 % per weight (-) epicatechin and 5-66%, preferably 7-25 %, more preferably 10-20 % per weight myricetin.
- Said composition additionally comprising 0,5-20%, preferably 1-10 %, more preferably 1,5-5 % per weight 4-hydroxyisoleucine.
- One of said compositions, wherein at least one ingredient is derived at least in part from plants or plant fractions.
- One of said compositions, wherein the hesperetin is present at least in part in form of orange extract (Citrus aurantium) and/or the (-) epicatechin is present at least in part in form of green tee extract (Camellia sinesis) and/or the (-) epicatechin is present at least in part in form of cocoa extract and/or the myricetin is present at least in part in form of wild bayberry extract and/or the 4-hydroxyisoleucine is present at least in part in form of fenugreek extract, if 4-hydroxyisoleucine is a component of the composition.
- One of said compositions, wherein the orange extract contains 30-100%, more preferably 50-95 %, even more preferably 75-90 % per weight hesperidin and/or the green tea extract (Camellia sinesis) and/or the cocoa contain 30-100%, more preferably 50-95 %, even more preferably 75-90 % per weight (-) epicatechin and/or the wild bayberry extract contains 25 - 100 %, more preferably 40 - 60 %, even more preferably 45-55 % per weight myricetin and/or the fenugreek extract contains 1 - 100 %, more preferably 10 - 60 %, even more preferably 15 - 25 % per weight 4-hydroxyisoleucine, if 4-hydroxyisoleucine is a component of the composition.
- One of said compositions additionally comprising nutrients, preferably magnesium chloride and/or magnesium citrate and/or magnesium lactate and/or vitamin D3.
- One of said compositions additionally comprising one or more flavonoids or its derivatives, preferably selected from the group consisting of kaempferol, leucodelphinidin, poncirin, diosmetin, taxifolin, dihydromyricetin, epigallocatechin-3-o-gallate, and/or genistein.
- One of said compositions additionally comprising silk peptide and/or leucine and/or β-hydroxy-β-methylbutyric acid and/or piperine.
- One of said compositions additionally comprising one or more excipients, preferably in form of hypromellose.
- A method for preventing muscle atrophy in a subject comprising the step of administering to said subject a composition as described before.
- Said method, wherein one of said compositions are administered orally.
- One of said methods, wherein one of said composition is administered in form of capsules or tablets.
- One of said methods, wherein one of said compositions is administered as part of dietary supplement or nutritional product.
- Use of one of said compositions as inhibitor for myostatin/ActRIIB signaling pathway.
- Use of one of said compositions as stimulator for PI3K/Akt signaling pathway.

## Claims

1. A composition comprising hesperetin, (-) epicatechin and myricetin.

2. Composition according to claim 1 comprising 15-66 % per weight hesperetin.

3. Composition according to claim 1 or 2 comprising 15-66 % per weight (-) epicatechin.

4. Composition according to any one of claims 1 to 3 comprising 5-66 % per weight myricetin.

5. Composition according to any one of claims 1 to 4 additionally comprising 0,5-20 %, per weight 4-hydroxyisoleucine.

6. Composition according to any one of claims 1 to 5,wherein at least one ingredient is derived at least in part from plants or plant fractions.

7. Composition according to any one of claims 1 to 6, wherein the hesperetin is present at least in part in form of orange extract (Citrus aurantium) and/or the (-) epicatechin is present at least in part in form of green tee extract (Camellia sinesis) and/or the (-) epicatechin is present at least in part in form of cocoa extract and/or the myricetin is present at least in part in form of wild bayberry extract and/or the 4-hydroxyisoleucine is present at least in part in form of fenugreek extract, if 4-hydroxyisoleucine is a component of the composition.

8. Composition according to claim 7, wherein the orange extract contains 30-100 %, per weight hesperidin and/or the green tea extract (Camellia sinesis) and/or the cocoa contain 30-100 % per weight (-) epicatechin and/or the wild bayberry extract contains 25 - 100 % per weight myricetin and/or the fenugreek extract contains 1 - 100 % per weight 4-hydroxyisoleucine, if 4-hydroxyisoleucine is a component of the composition.

9. Composition according to any one of claims 1 to 8 additionally comprising nutrients, preferably magnesium chloride and/or magnesium citrate and/or magnesium lactate and/or vitamin D3.

10. Composition according to any one of claims 1 to 9 additionally comprising one or more flavonoids or its derivatives, preferably selected from the group consisting of kaempferol, leucodelphinidin, poncirin, diosmetin, taxifolin, dihydromyricetin, epigallocatechin-3-o-gallate, and/or genistein.

11. Composition according to any one of claims 1 to 10 additionally comprising silk peptide and/or leucine and/or β-hydroxy-β-methylbutyric acid and/or piperine.

12. Composition according to any one of claims 1 to 11 additionally comprising one or more excipients, preferably in form of hypromellose.

13. Use of the composition according to any one of claims 1 to 12 as a dietary supplement or nutritional product.

14. Use of the composition according to any one of claims 1 to 12 as inhibitor for myostatin/ActRIIB signaling pathway.

15. Use of the composition according to any one of claims 1 to 12 as stimulator for PI3K/Akt signaling pathway.
